Europäisches Patentamt

European Patent Office

Office européen des brevets

(11) Veröffentlichungsnummer: **0 045 863**

**A2**

(12) # EUROPÄISCHE PATENTANMELDUNG

(21) Anmeldenummer: **81105644.9**

(22) Anmeldetag: **18.07.81**

(51) Int. Cl.³: **A 61 B 5/14**
**B 01 L 3/02**

(30) Priorität: **07.08.80 DE 3029886**

(43) Veröffentlichungstag der Anmeldung:
**17.02.82 Patentblatt 82.7**

(84) Benannte Vertragsstaaten:
**AT BE CH DE FR GB IT LI LU NL SE**

(71) Anmelder: **C.A. Greiner & Söhne GmbH & Co. KG**
**Galgenbergstrasse 9c**
**D-7440 Nürtingen(DE)**

(72) Erfinder: **Feaster, William Wardock**
**L'Estoril Avenue Princesse Grace**
**Monte Carlo(MC)**

(74) Vertreter: **Dreiss, Uwe, Dr. jur. Dipl.-Ing. M.Sc. et al,**
**Patentanwälte Dreiss, Hosenthien & Fuhlendorf**
**Gerokstrasse 6**
**D-7000 Stuttgart 1(DE)**

(54) **Blutentnahmegerät.**

(57) Die Erfindung geht von einem Blutentnahmesystem aus, mit dem in einen einseitig offenen auf der anderen Seite mit einer doppelendigen Hohlnadel versehenen zylindrischen Halter ein Blutentnahmeröhrchen eingeschoben wird, das evakuiert und mit einer durchstechbaren Membran verschlossen ist, so daß beim Einschieben das in das Innere des Halters hineinragende Ende der doppelendigen Hohlnadel die Membran durchsticht und so eine Verbindung des evakuierten Inneren des Blutprobenröhrchens mit dem äußeren Ende der Hohlnadel herstellt. Zur erleichterung des Durchstichs des inneren Endes der Hohlnadel durch die Membran bei exzentrischer Anordnung der Nadel im Halter weist die Membran eine Ringnut (219) auf, die eine Dünnstelle (519') ergibt, auf die die Nadel beim Einschieben des Blutprobenröhrchens in den Halter trifft. Die Membran weist eine weitere Dünnstelle (520') auf, die mit einer Pipette durchstochen werden kann.

Fig.1

EP 0 045 863 A2

## Blutentnahmegerät

Die Erfindung betrifft ein Blutentnahmegerät mit einem einseitig offenen hohlzylindrischen Halter und einem in dessen offenes Ende einschiebbaren Blutprobenröhrchen, wobei in das geschlossene Ende des Halters eine doppelendige hohle Nadel exzentrisch eingeschraubt ist und das Blutprobenröhrchen durch eine eine Öffnung aufweisende Kappe und eine die Öffnung verschließende Dichtungsscheibe abgedichtet ist.

Ein derartiges Blutentnahmegerät ist in der europäischen Patentanmeldung 80 100 830.1 beschrieben.

Aufgabe der vorliegenden Erfindung ist es, ein solches Blutentnahmegerät derart weiterzubilden, daß ein leichteres Durchstechen der Dichtungsscheibe ohne Gefährdung ihrer Abdichtung und Halterung möglich ist und das Blut auf besonders einfache Weise entnommen werden kann.

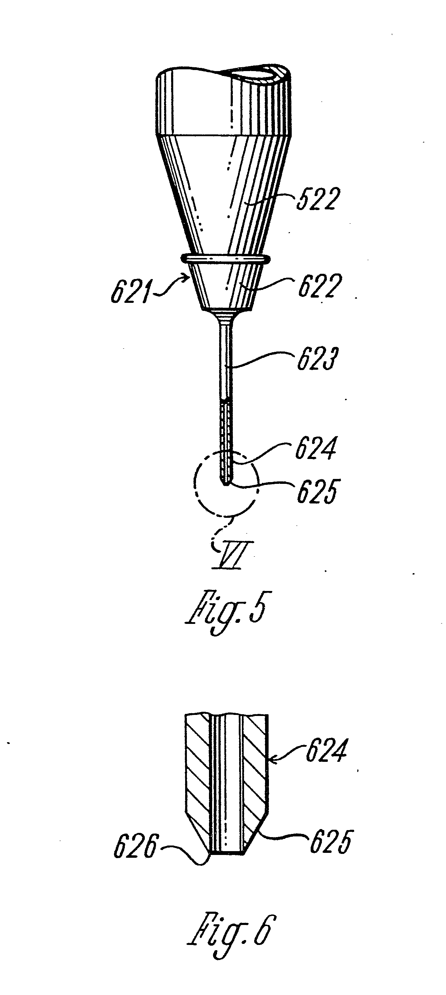

Diese Aufgabe wird dadurch gelöst, daß die Membran die Form einer kreisförmigen Nut aufweist, deren Durchmesser gleich dem Abstand der Nadel von der Mittelachse des Halters ist. Es wird also ein ringförmiger verdünnter Bereich geschaffen, der sowohl bei exzentrischer Anordnung der Nadel als auch bei Anordnung von zwei Nadeln im Halter gegenüber dem in das Innere des Halters hineinragenden Ende der Nadel angeordnet ist. Die Nadel trifft somit auf diesen verdünnten Bereich und kann daher besonders leicht durch die Membran hindurchgestoßen werden. Wichtig ist hierbei also, daß nur ein in radialer Richtung relativ kleiner Bereich, also die Ringnut, eine Schwachstelle zum Durchstechen des inneren Endes der doppelendigen Nadel bildet, um so die Dichtwirkung und die mechanische Haltbarkeit der Nadel nur minimal zu schwächen. Insofern unterscheidet sich der Gegenstand der vorliegenden Anmeldung von einer Ausbildung gemäß Fig. 6 der genannten älteren Anmeldung, bei der die Ausnehmung großflächig der Größe der gesamten Öffnung in der Kappe entspricht und praktisch dadurch entsteht, daß die zwischen Kappe und Blutprobenröhrchen eingedrückten Ränder der Dichtungsscheibe besonders dick ausgebildet sind. Die Ringnut eignet sich sowohl für Blutentnahmegeräte der genannten Art mit einer exzentrischen Nadel, weil es dann beim Ansetzen der Nadel nicht darauf ankommt, die Nadel in einer genau bestimmten Position auf das Blutprobenröhrchen zu schieben; die Ringnut ist aber auch besonders geeignet für Blutentnahmegeräte, bei denen eine weitere Nadel in das Innere des Halters hineinragt und die Verbindung mit einer Unterdruckquelle herstellt, durch die das Röhrchen evakuiert wird. Haben sowohl die erste wie auch die zweite Nadel von der Mittelachse des Halters einen bestimmten Abstand und ist dieser Abstand gleich dem Radius der Ringnut in der Dichtungsscheibe, so treffen beide Nadeln auf die in der Membran durch die Ringnut geschaffenen Dünnstellen, so daß dies eine besonders einfache Weiterbildung der 2-nadeligen Ausführung der Erfindung nach Fig. 4b der genannten Patentanmeldung darstellt.

Eine Weiterbildung der Erfindung sieht vor, daß eine weitere Ausnehmung vorgesehen ist. Dadurch kann man besonders einfach aus dem Blutprobenröhrchen nach der Blutentnahme Blut entnehmen, um es weiteren Analysen zuzuführen. Diese weitere Ausnehmung, vorzugsweise in der Mitte der Dichtungsscheibe, hat einen weiteren verdünnten Bereich, der von einer Pipettenspitze leicht durchstoßen werden kann. Es ist also möglich, ohne Abschrauben des aus Kappe und Membran gebildeten Verschlusses Blut aus dem Blutprobenröhrchen zu entnehmen.

Praktisch ist durch diese Weiterbildung der Erfindung erstmalig ein vollkommen abgeschlossenes System geschaffen worden, bei dem das Blut sowohl direkt von der Vene in ein verschlossenes Blutprobenröhrchen überführt als auch aus diesem direkt ohne Abnehmen des Verschlusses in eine Pipette überführt wird. Damit ist die Ansteckungsgefahr, die Gefahr von Verunreinigungen, auch wechselseitiger Verunreinigung (Cross-Contamination), auf ein absolutes Minimum herabgesetzt.

Ausführungsbeispiele der Erfindung werden im folgenden anhand der beigefügten Zeichnungen beschrieben. Es stellen dar:

Figur 1 ein Ausführungsbeispiel;
Figur 2 eine Draufsicht auf die im Ausführungsbeispiel verwendete Dichtungsscheibe;
Figur 3 die Entnahme von Blut mit Hilfe einer Pipette aus einem Blutprobenröhrchen nach Fig. 1;
Figur 4 eine vergrößerte Darstellung des Bereiches IV in Fig. 3;
Figur 5 eine Pipettenspitze;
Figur 6 eine vergrößerte Darstellung des Bereiches VI in Fig. 5.

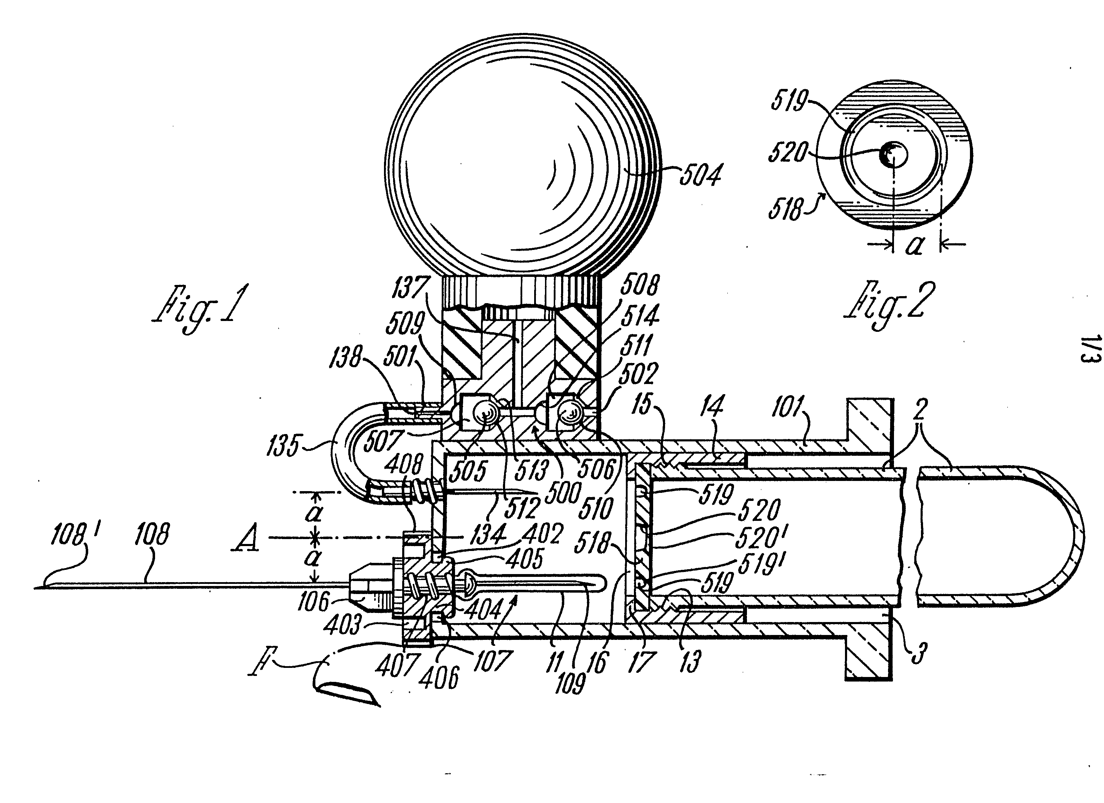

Das Blutentnahmegerät nach Fig. 1 wird durch einen Halter 101 und ein Blutprobenröhrchen 2 gebildet.

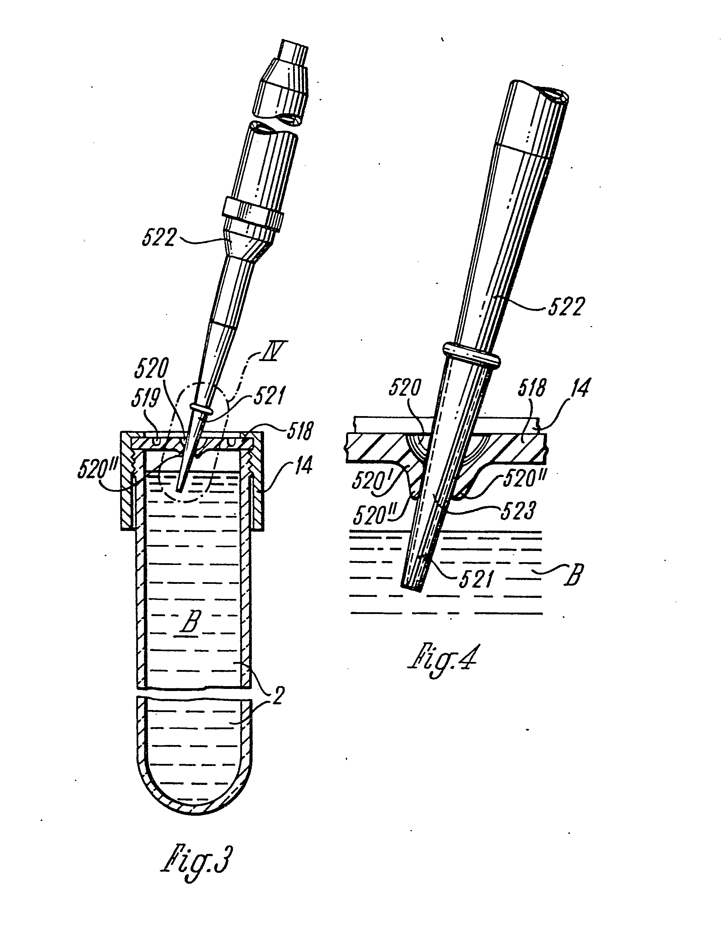

Der Halter 101 ist hohlzylindrisch und an seinem Ende 3 offen. An seinem anderen Ende ist eine doppelendige hohle Nadel 107 eingeschraubt, deren inneres Ende 107' von einer Schutzhülle 11 umgeben ist. Das Blutprobenröhrchen 2 weist an seinem linken Ende ein Schraubgewinde 13 auf, auf das eine mit einem Innengewinde 15 versehene Kappe 14 aufgeschraubt ist. Die Kappe 14 weist eine Öffnung 16 auf. Deren Durchmesser ist so bestimmt, daß der Rand 17 der Kappe 14, der sich von außen nach innen senkrecht zur Achse des Blutprobenröhrchens erstreckt, eine Dichtungsscheibe 518 gegen die (in Fig. 1) linke Stirnseite des Blutprobenröhrchens 2 drückt und damit eine Dichtung bildet. Kurz vor einer Blutentnahme erzeugt man im Innern des Blutprobenröhrchens 2 ein Vakuum. Das erfolgt dadurch, daß die Dichtungsscheibe von einer (nicht gezeigten) Nadel durchstochen wird, die an eine (nicht gezeigte) Unterdruckquelle angeschlossen ist (vgl. dazu Fig. 3 der eingangs genannten europäischen Patentanmeldung). Bei der Blutentnahme wird dann zunächst das linke äußere Ende 107" der Nadel 107, die in der gezeigten Weise in den Halter 101 eingeschraubt ist, in die Vene eines Patienten eingestochen. Dann wird das Blutprobenröhrchen 2 so weit (in Fig. 2: nach links) in den Halter 101 eingeschoben, daß das rechte Ende 107' der Nadel 107 die Membran 518 durchsticht. Damit ist eine Verbindung des äußeren Endes 107", das in eine Vene eingestochen ist, mit dem evakuierten Innenraum des Blutprobenröhrchens 2 hergestellt; sodann kann das Vakuum über die Nadel Blut aus der Vene ansaugen.

In den Halter 101 ist eine weitere Nadel 134 eingeschraubt, die mit einer Hohlleitung 135 in Verbindung steht. Diese Hohlleitung 135 ist über eine Steckver-

bindung 138 mit einer Öffnung 501 eines Dreiwegeventils 500 verbunden; das Dreiwegeventil 500 weist außerdem eine weitere Öffnung 502, die ins Freie geht, sowie eine Saugleitung 137, die die Verbindung mit einem Gummiballon 504 herstellt, auf. Wird der Gummiballon 504 zusammengedrückt, so entsteht in der Saugleitung 137 Druck. Die Kugel 505 in der linken Kammer 507 wird nach links auf den Ventilsitz 509 gedrückt und verschließt diesen. Somit kann keine Luft in die Hohlleitung 135 austreten. Gleichzeitig wird die rechte Kugel 506 in der Kammer 508 auf den rechten Ventilsitz 510 gedrückt. Dieser weist jedoch Schlitze 511 auf. Durch diese Schlitze und die Öffnung 502 kann die Luft ins Freie austreten. Läßt man den zusammengedrückten Gummiballon 504 los, zieht der entstehende Unterdruck die Kugel 505 nach rechts und damit auf den Ventilsitz 512. Doch ist der Ventilsitz 512 damit nicht verschlossen, da in ihm Schlitze 513 vorgesehen sind. Luft kann also von der Hohlleitung 135 über die Öffnung 501 von der Saugleitung 137 her in den Gummiballon 504 eintreten. Dabei verschließt die Kugel 506, die nach links gezogen wird, den Ventilsitz 514. Bei Loslassen des zusammengedrückten Gummiballons 504 wird also der dabei in diesem entstehende Unterdruck über die Saugleitung 137, die Schlitze 512, die Kammer 507, die Hohlleitung 135 und die Nadel 134 in dem Blutprobenröhrchen 2 wirksam. Ist nun das Blutprobenröhrchen 2 so weit nach links im Halter 101 geschoben, daß sowohl das rechte Ende 107' der Nadel 107 als auch die Nadel 134 die Membran 518 durchstoßen, so wird dann auf diese Weise der durch Zusammenpressen des Gummiballons 504 entstandene Unterdruck nicht nur im Blutprobenröhrchen 2, sondern auch in der Nadel 107 wirksam und saugt so aus der Vene eines Patienten Blut an. Der mit Hilfe des Gummiballons 504 erzeugte Unterdruck kann also je nach Betätigung den sowieso schon vorhandenen Unterdruck im Blutprobenröhrchen 2 unterstützen.

In der Membran 518 ist eine ringförmige Nut 519 vorgesehen. Ihr Radius a ist gleich dem Abstand der Nadeln 107 und 134 von der Mittelachse A des Halters 101. Damit ist gewährleistet, daß beim Einschieben des Blutprobenröhrchens 2 in den Halter 101 das Ende 107' der Nadel 107 und auch die Nadel 134 genau in diese Nut 519 treffen, so daß sie nur den verdünnten Bereich (Dünnstelle) 519' am Boden der Nut 519 durchstoßen müssen.

Aus der Tatsache, daß die Nadeln 107 und 134 im Halter 101 exzentrishc angeordnet sind, ergibt sich, daß die Nut 519, auch wenn nur die Nadel 107 vorgesehen wäre, als ringförmige Nut vorgesehen werden muß, damit in jeder Drehstellung von Blutprobenröhrchen 2 und Halter 101 zueinander das Ende 107' der Nadel 107 auf einen verdünnten Bereich 519' der Membran 518 treffen kann.

Ferner weist die Membran 518 eine mittlere Ausnehmung 520 auf. An ihrem Boden entsteht ein verdünnter Bereich 520'. Ihr Zweck ist aus Fig. 3 ersichtlich. Die Ausnehmung 520 kann von einer Pipettenspitze 521, die auf einer Pipette 522 aufgesetzt ist, durchstoßen werden. Dann legen sich die Ränder 520', wie aus Fig.4 ersichtlich, an den Rand der Pipettenspitze 520 an. Derart kann aus dem noch durch Kappe 514 und Dichtungsscheibe 518 verschlossenen Blutprobenröhrchen 2 Blut entnommen werden.

Auf diese Weise entsteht ein vollkommen abgeschlossenes Blutentnahmesystem, bei dem erstens direkt aus der Vene eines Patienten Blut in ein Blutprobenröhrchen angesaugt wird und zweitens wieder entnommen werden kann. Beides erfolgt, ohne daß man den Verschluß (bestehend aus Kappe 514 und Dichtungsscheibe 518) des Blutprobenröhrchens 2 öffnen muß. Auf diese Weise ist die Gefahr einer Verunreinigung der Probe, einer Ansteckung durch in der Probe enthaltene Krankheitserreger (insbesondere z.B. bei Hepatitis), sowie einer gegenseitigen Verunreinigung mehrerer Proben (Cross- Contamination) gegenüber sämtlichen seither bekannten Systemen auf ein absolutes Minimum heruntergedrückt.

Als Pipettenspitzen 521 kommen herkömmliche Pipettenspitzen, wie sie auf dem Markt erhältlich sind, in Betracht. Es ist aber auch möglich, den Pipettenspitzen im Hinblick auf die Verwendung im Zusammenhang mit dem hier beschriebenen Blutentnahmesystem eine im vorderen Bereich besonders dünne, zylindrische und spitze, d.h. eine dieser Anwendung besonders angepaßte Form zu geben, so daß die Durchstichstelle zwischen den Rändern 520' möglichst geringen Durchmesser hat, so daß sie sich nach Herausziehen der Pipettenspitze wieder vollständig schließt. Eine solche Form ist in Fig. 5 dargestellt. Die dort gezeigte Pipettenspitze 621, die auch mit einer Pipette 522 verwendbar ist, weist zunächst einen konischen Bereich 622 auf, mit dem sie auf eine Pipette 522 aufsetzbar ist. Dieser konische Bereich geht in einen zylindrischen Bereich 623 mit sehr kleinem Durchmesser über. Daher kann man durch den Bereich 520' am Boden der Ausnehmung 520 sehr weit hindurch stechen, ohne daß die entstehende Durchstichöffnung 523 zu weit aufgeweitet wird.

- 8 -

Die Durchstichöffnung 523 zwischen den Rändern 520" wird also beim Durchstechen nicht sher groß und kann sich nach Herausnehmen der Pipettenspitze wieder schließen. Dabei wird dann beim Herausziehen der Pipettenspitze aus der Durchstichöffnung die Außenseite abgewischt und an den Rändern 520" abgestreift. Außerdem ist die Spitze 624 an ihrem vorderen Ende mit einer Abschrägung 625 versehen, so daß am vorderen Ende der Pipettenspitze eine Schneidkante 626 entsteht, die den Durchstich durch den verdünnten Bereich 520' erleichtert.

- Ende der Beschreibung -

Patentansprüche

1. Blutentnahmegerät mit einem einseitig offenen hohlzylindrischen Halter (10) und einem in dessen offenes Ende (3) einschiebbaren Blutprobenröhrchen (2), wobei in das geschlossene Ende des Halters (101) eine doppelendige hohle Nadel (108) exzentrisch eingeschraubt ist und das Blutprobenröhrchen (2) durch eine eine Öffnung (16) aufweisende Kappe (14) und eine die Öffnung (16) verschließende Dichtungsscheibe (518) abgedichtet ist, dadurch gekennzeichnet, daß die Membran (518) die Form einer kreisförmigen Nut (519) aufweist, deren Durchmesser (a) gleich dem Abstand der Nadel (107) von der Mittelachse (A) des Halters (101) ist.

2. Blutentnahmegerät nach Anspruch 1, dadurch gekennzeichnet, daß eine weitere Ausnehmung (520) im Zentrum der Membran (518) vorgesehen ist, wobei die Ausnehmung (520) so tief ist, daß der verbleibende verdünnte Bereich (520') am Boden der Ausnehmung durch eine Pipettenspitze (521, 621) durchstechbar ist.

3. Pipettenspitze zur Blutentnahme aus einem Probenröhrchen des Blutentnahmegerätes nach Anspruch 2, dadurch gekennzeichnet, daß die Pipettenspitze in an sich bekannter Weise einen konischen Teil (622) zum Aufsetzen auf eine Pipette (522) aufweist und daß sich an diesen ein zylindrischer Teil (623) mit geringerem gleichbleibendem Durchmesser anschließt.

4. Pipettenspitze nach Anspruch 3, dadurch gekennzeichnet, daß das vordere Ende der Pipettenspitze mit einer Schneidkante (626) versehen ist.

- Ende der Ansprüche -

*Fig.1*

*Fig.2*

0045863

0045863

522
520
519
520''
521
518
14
520''
IV
B
2

*Fig.3*

522
520
520'
520''
520''
518
14
520''
523
521
B

*Fig.4*

522

621 622

623

624

625

$\overline{VI}$

*Fig.5*

624

626 625

*Fig.6*